(19) Europäisches Patentamt
European Patent Office
Office européen des brevets

(11) **EP 2 785 314 B2**

(12) **NOUVEAU FASCICULE DE BREVET EUROPEEN**
Après la procédure d'opposition

(45) Date de publication et mention de la décision concernant l'opposition:
**04.11.2020 Bulletin 2020/45**

(45) Mention de la délivrance du brevet:
**14.02.2018 Bulletin 2018/07**

(21) Numéro de dépôt: **12797837.7**

(22) Date de dépôt: **28.11.2012**

(51) Int Cl.:
*A61K 8/365* (2006.01)   *A61K 8/97* (2017.01)
*A61K 31/185* (2006.01)   *A61Q 19/08* (2006.01)
*A61K 35/748* (2015.01)

(86) Numéro de dépôt international:
**PCT/EP2012/073860**

(87) Numéro de publication internationale:
**WO 2013/079546 (06.06.2013 Gazette 2013/23)**

(54) **COMPOSITION TOPIQUE COMPRENANT UNE ASSOCIATION D'AU MOINS UN EXTRAIT D'ALGUE BLEUE AVEC AU MOINS UN ACIDE ALPHA HYDROXYLE OU UN DE SES SELS**

TOPISCHE ZUSAMMENSETZUNG MIT EINER KOMBINATION AUS MINDESTENS EINEM BLAUALGENEXTRAKT MIT MINDESTENS EINER ALPHAHYDROXYSÄURE ODER EINEM IHRER SALZE

TOPICAL COMPOSITION CONTAINING A COMBINATION OF AT LEAST ONE BLUE ALGAE EXTRACT WITH AT LEAST ONE ALPHA HYDROXY ACID OR ONE OF THE SALTS THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.11.2011 FR 1160853**

(43) Date de publication de la demande:
**08.10.2014 Bulletin 2014/41**

(73) Titulaire: **Pierre Fabre Dermo-Cosmétique**
**92100 Boulogne-Billancourt (FR)**

(72) Inventeurs:
• **BRUNEL, Yves**
**F-81150 Marssac-sur-Tarn (FR)**
• **CASTEX-RIZZI, Nathalie**
**F-31770 Colomiers (FR)**
• **DUBOIS, Jacques**
**F-11100 Narbonne (FR)**

(74) Mandataire: **Regimbeau**
**20, rue de Chazelles**
**75847 Paris Cedex 17 (FR)**

(56) Documents cités:
**EP-A1- 0 385 841      EP-A2- 0 273 202**
**DE-A1- 10 059 107      FR-A1- 2 787 711**
**FR-A1- 2 876 030**

EP 2 785 314 B2

**Description**

La présente invention concerne le domaine des algues bleues et leur utilisation associée à au moins un acide alpha hydroxylé ou un de ses sels dans une composition destinée à un usage topique, de nature pharmaceutique ou cosmétique.

Les algues bleues (ou cyanophycées, ou cyanobactéries) sont des procaryotes photosynthétiques qui utilisent l'énergie lumineuse comme source d'énergie. Leur information génétique est portée par une molécule cyclique d'acide désoxyribonucléique et des plasmides. L'oxydation de l'eau en oxygène au cours de la photosynthèse est une propriété qui les sépare totalement des autres procaryotes photosynthétiques, les bactéries pourpres et vertes.

Le nom d'algues bleues est dû à la présence de phycocyanines. Si l'on extrait les pigments solubles dans l'acétone (chlorophylles et caroténoïdes) d'une culture d'algues bleues la couleur bleue devient alors parfaitement visible.

Leur couleur résulte de la présence de ces pigments (chlorophylles vertes, carotènes rouges et oranges, xanthophylles jaunes, phycocyanines bleues et phycoérythrines rouges) et de mucilage.

Les algues bleues absorbent surtout dans le bleu et dans le vert. Ces longueurs d'onde sont actives pour la photosynthèse. L'absorption maximale se réalise dans le bleu (< 500nm) ce qui permet aux algues bleues de tirer une plus grande énergie chimique de l'énergie lumineuse absorbée.

Les algues bleues constituent un groupe génétiquement et morphologiquement très hétérogène. Elles présentent une grande diversité morphologique. Certaines sont unicellulaires, sphériques ou en bâtonnets et se multiplient par divisions binaires, d'autres sont filamenteuses et se propagent par rupture du filament végétatif ou par germination des akinètes (spores).

Cette hétérogénéité leur a permis de coloniser la grande majorité des écosystèmes terrestres ou aquatiques y compris les milieux extrêmes chauds, salés et hyper salés et alcalins. Il existe des formes terrestres, aquatiques, libres et symbiotiques.

Dès 1962 une publication (Lefèvre/Laporte : Cyanostimulines et cicatrisation) décrivait les effets étonnants du jus d'algue bleu *Phormidium Uncinatum*, issue de bassins thermaux de Bagnères de Bigorre en France, sur la prolifération cellulaire (FR1345584). Des études cliniques démontraient l'accélération de la cicatrisation de plaies par des emplâtres de cette algue (FR2674M).

Les acides alpha hydroxylés (AAH) sont des acides carboxyliques portant une fonction hydroxyle (-OH)) sur le carbone adjacent du groupe carboxylique. Ces acides ont trouvé un large éventail d'applications dans l'industrie cosmétique comme elles se sont révélées être sans danger sur la peau. En raison de leur caractère acide, ils sont utilisés comme agent exfoliant pour le lissage de la peau. Cette propriété des acides alpha-hydroxylés est particulièrement utilisée en cosmétique pour retarder l'apparition des rides.

Les principaux AAH qui sont actuellement utilisés dans l'industrie cosmétique sont l'acide lactique, l'acide glycolique, et l'acide citrique mais il en existe d'autres.

Les acides alpha-hydroxylés comprennent, sans s'y limiter, les composés suivants : l'acide lactique (50-21-5), l'acide glycolique (79-14-1), l'acide citrique (77-92-9), l'acide malique (6915-15-7), l'acide alpha-hydroxyéthanoïque, l'acide alpha-hydroxyoctanoïque, l'acide alpha-hydroxycaprylique, , les acides mixtes de fruits, les triples acides de fruits, les tri-alpha hydroxy acides de fruits, les complexes d'alpha hydroxy d'extraits de plantes, le L-alpha hydroxy acide et le glycomère d'acides gras réticulés.

- Acide lactique :

L'acide lactique est utilisé comme agent exfoliant (pour les peaux sensibles), il peut agir comme un agent antibactérien, il entre dans la composition du film hydro-lipidique cutané et contribue ainsi au maintien du pH de la peau, il évite ainsi les infections bactériennes et participe à l'hydratation de la peau. L'acide lactique a la propriété d'améliorer l'absorption de cosmétiques tels que les lotions solaires.

- Acide Glycolique :

Cet acide a le plus faible poids moléculaire dans la catégorie des AAH. C'est un solide cristallin, soluble dans l'eau. Il est largement utilisé dans divers produits de soins de la peau. L'application la plus courante de cet acide concerne les peelings chimiques à une concentration de 20 à 70% lorsqu'ils sont prescrits et appliqués par les dermatologues ou jusqu'à 10% dans les produits destinés à l'usage des particuliers. L'acide glycolique est indiqué dans le traitement de l'acné, des rides, de l'hyper-pigmentation et de l'hyperkératose. Lorsqu'il est utilisé dans la kératose, son mécanisme d'action consiste à affaiblir la liaison de lipides dans les cellules mortes de la peau, à diminuer la cohésion intercellulaire et, au final, à exfolier la couche cornée

- Acide citrique :

L'acide citrique est présent dans les fruits comme les citrons, les oranges et les pamplemousses. L'acide citrique trouve des applications dans de nombreux anti-vieillissements ou crèmes anti-rides. Il est également un bon astringent et aide dans le traitement des taches disgracieuses. Il améliore également l'efficacité des principes hydratants pour la peau. L'acide citrique est également utilisé dans les produits capillaires.

Les compositions selon l'invention s'étendent également à celles contenant d'autres sels d'acides organiques et en particulier aux sels suivants :

- L'acétate (sel d'acide acétique)
- Le propionate (sel d'acide propionique)
- Le butyrate (sel d'acide butyrique)
- Le pyruvate (sel d'acide pyruvique)
- Le formate (sel d'acide formique)
- Le tartrate (sel d'acide tartrique)
- Le malate (sel d'acide malique)
- Le fumarate (sel d'acide fumarique)
- Le sorbate (sel d'acide sorbique)

Les travaux à la base de l'invention ont tout particulièrement porté sur l'association d'algue bleue et de sels d'acide lactique, mais les algues bleues peuvent être associées à tout autre acide alpha hydroxylé ou un de ses sels.

L'acide lactique (acide 2-hydroxypropanoïque) est un acide organique qui joue un rôle dans divers processus biochimiques. C'est un acide carboxylique hydroxylé, de formule chimique $C_3H_6O_3$. L'acide lactique est présent dans le lait, mais également dans le vin, certains fruits et légumes, et dans les muscles. Un lactate est un sel de cet acide.

L'acide lactique se présente aussi sous forme de sels : sel de sodium, de potassium, de magnésium et de calcium. Ses sels sont sous forme de poudre et sont également solubles en milieu aqueux. Il agit comme agent bactériostatique notamment sur des bactéries pathogènes comme la salmonelle (ou la listeria), mais c'est surtout pour leurs propriétés humectantes et hydratantes que les lactates sont utilisé en cosmétique depuis longtemps.

En plus de leurs propriétés hydratantes incontestables les lactates agissent aussi comme de bons tampons qui aident à stabiliser le pH et aide à maintenir la stabilité microbienne des formulations, due à ces propriétés bactériostatiques évoquées précédemment.

La peau est formée de trois couches, l'hypoderme, le plus profond, le derme et l'épiderme. Ce dernier est un épithélium malpighien kératinisé qui protège le corps des agressions mécaniques, chimiques et biologiques, empêche les pertes hydriques en limitant l'évaporation de l'eau contenue dans la peau et participe à la photo-protection en adsorbant une partie des rayons ultra-violets. Ces fonctions vitales sont appelées collectivement «fonctions de barrière épidermique».

L'épiderme est constitué majoritairement de kératinocytes. Ceux-ci prolifèrent au niveau de la couche basale puis subissent un programme de différenciation vectorisé pour constituer successivement les couches épineuse puis granuleuse. Finalement, au cours de ce processus de kératinisation, les cellules dégénèrent et se transforment en cornéocytes. L'accumulation des cornéocytes forme la couche cellulaire la plus externe de l'épiderme, appelée couche cornée ou *stratum corneum.* Le *stratum corneum* est le principal responsable des fonctions de barrière épidermique, en raison de sa résistance mécanique, de son étanchéité et de son contenu.

Le derme est un tissu de type conjonctif formant la peau avec l'épiderme et l'hypoderme. Son épaisseur est variable selon les régions corporelles.

Son caractère conjonctif vient de sa composition :

Il contient des macromolécules de type protéique, en particulier fibres de collagène, élastine et de fibronectine conférant à la peau souplesse, élasticité et assise. Ces fibres protéiques font de lui une véritable assise pour l'épiderme dont le vieillissement est à l'origine de l'apparition des rides et autres signes du vieillissement cutané.

Le derme est également constitué de mucopolysaccharides ou glycosaminoglycanes, protéines qui à la manière d'une éponge vont capter l'eau dans le derme et ainsi agir comme réservoir d'hydratation.

Enfin le derme contient diverses cellules dont les fibroblastes et les cellules du système immunitaire.

Le derme est irrigué par le sang à travers un important système vasculaire. Il prend en charge la nutrition de l'épiderme par diffusion.

Outre son rôle nutritif, le derme joue également un rôle primordial dans la thermorégulation et dans la cicatrisation grâce aux nombreux échanges qui existent entre le derme et l'épiderme. Les liens entre ces deux constituants de notre peau ont pu être démontrés à travers la présence de récepteurs épidermiques dans le derme tel que CD44 et les nombreux phénomènes de régulation et d'interaction qui existent entre ces deux tissus.

La peau est l'élément du corps humain qui est le plus en contact avec l'environnement extérieur.

Malgré les travaux réalisés à ce jour, le vieillissement cutané est encore mal connu.

Le vieillissement cutané est déterminé par des facteurs génétiques et environnementaux.

On distingue 2 types de vieillissement :

Le vieillissement intrinsèque ou chronologique correspond aux changements inévitables liés à l'âge; la ménopause ou plus précisément la carence en œstrogènes accélère le vieillissement cutané chronologique. Il affecte la peau comme les autres organes.

Il correspond aux modifications observées sur les zones protégées du soleil chez tous les individus mais avec des variations entre les personnes qui sont génétiquement déterminées.

Ce type de vieillissement se caractérise par :

- une sécheresse de la peau souvent à l'origine de démangeaisons
- une réduction de l'élasticité cutanée
- une diminution de l'épaisseur cutanée (atrophie du derme et de l'épiderme)
- des ridules et de fines rides
- réduction de la vascularisation du derme et ralentissement de l'activité métabolique.

une fragilité cutanée et capillaire à l'origine d'ecchymoses et de plaies au moindre traumatisme.

Avec le vieillissement, on observe une réduction de la sudation et de l'hydratation cutanée du fait d'une réduction du nombre de follicules pileux et des glandes annexes de la peau.

Le vieillissement extrinsèque est lié aux facteurs de l'environnement, notamment le soleil. Le terme de vieillissement actinique ou héliodermie correspond à des modifications caractéristiques de la peau liées à l'exposition solaire chronique et siège sur les zones photo-exposées.

Sur les zones de la peau exposées au soleil (visage, dos des mains, avant-bras) apparaissent des tâches pigmentées, des rides et des rougeurs.

Si les dommages s'intensifient, on observe un épaississement de l'épiderme et la peau apparaît jaunâtre, plus sèche, les rides se creusent ;la peau présente une pigmentation irrégulière avec des tâches brunes et blanches.

Il peut exister aussi des cicatrices blanchâtres en forme d'étoile (pseudo-cicatrice stellaire) en particulier sur les avant-bras à la suite de traumatismes minimes.

D'autres facteurs interviennent : le tabac, la consommation d'alcool et une mauvaise alimentation.

Le vieillissement cutané résulte de phénomènes complexes déterminés génétiquement et par des facteurs de l'environnement qui conduisent à des anomalies du génome et de l'organisation cellulaire et tissulaire.

L'élément déterminant du vieillissement cutané semble être la production d'espèces réactives de l'oxygène conduisant à un stress oxydatif.

Plusieurs études se sont intéressées au vieillissement cutané. Au cours du vieillissement cutané, on constate une altération du derme et de l'épiderme : diminution de la synthèse de collagène, altération des fibres élastiques, diminution de l'acide hyaluronique et de la vascularisation du derme.

De façon surprenante et inattendue, la demanderesse a montré qu'une composition contenant une association d'au moins un extrait d'algue bleue de type *Phormidium sp.* et d'au moins un sel d'acide alpha hydroxylé choisi parmi les sels de magnésium, de calcium et leurs mélangespermettait la prévention des signes de vieillissement et plus particulièrement le vieillissement cutané.

Par vieillissement cutané on entend notamment une perte d'élasticité des tissus (perte de la propriété à recouvrer son état initial après étirement), un changement de texture de la peau (épaississement ou amincissement), la diminution de la synthèse du collagène, la production d'espèces réactives de l'oxygène conduisant à un stress oxydatif et, en superficie, une sécheresse de la peau, l'apparition d'anomalies superficielles (rides, taches brunes, petits vaisseaux, réduction de la vascularisation et de la circulation du derme,...).

Cette prévention à pu être démontrée au travers de la stimulation de la sécrétion du facteur de croissance de l'endothélium vasculaire (aussi appelé VEGF) favorisant ainsi la revascularisation du derme et par conséquent la stimulation de la production naturelle de fibres de collagène et d'acide hyaluronique, l'inhibition d'enzymes dégradant la matrice extracellulaire (MMPs), et une activité anti-oxydante sur les fibroblastes.

La demanderesse a également montré qu'une composition contenant une association d'au moins un extrait d' algue bleue de type *Phormidium sp.* et d'au moins un sel d'acide alpha hydroxylé choisi parmi les sels de magnésium, de calcium et leurs mélanges permettait la prévention des signes de vieillissement cutané au travers de l'hydratation de l'épiderme, pour l'amélioration de toute forme de sécheresse cutanée ; pour le renforcement des fonctions de barrière de l'épiderme en favorisant la cicatrisation et la régénération de l'épiderme.

Cet effet a pu être démontré au travers de la stimulation de la migration et de la prolifération des kératinocytes, de la vascularisation par la production de VEGF et de la synthèse d'acide hyaluronique.

Par hydratation de l'épiderme on entend l'amélioration ou le maintien de l'équilibre en eau de l'épiderme.

Par l'amélioration de toute forme de sécheresse cutanée on entend toute amélioration de l'hydratation de l'épiderme notamment caractérisée par un manque d'eau dans la couche cornée, un film hydrolipidique situé à la surface trop fin et qui ne protège plus la peau, le manque de sébum.

Cet effet a pu être démontré au travers de la stimulation de la production d'acide hyaluronique et de son récepteur le CD44.

L'objet de la présente invention concerne donc une composition topique, pharmaceutique ou cosmétique, contenant au moins un extrait d'algue bleue de type *Phormidium sp.* et au moins un sel d'acide alpha hydroxylé choisi parmi les sels de magnésium, de calcium et leurs mélanges en association avec au moins un excipient acceptable pour l'application topique sur la peau et les phanères et les muqueuses.

De préférence la présente invention concernera une composition topique contenant au moins un extrait d'algue bleue de type *Phormidium sp.* et au moins un sel de lactate en association avec au moins un excipient permettant son application topique, en particulier un excipient pharmaceutiquement ou cosmétiquement acceptable.

L'algue bleue utilisée dans le cadre de l'invention est donc de type *Phormidium sp.*

Le sel d'acide organique biologique soluble en milieu aqueux pourra est choisi parmi : un sel de magnésium et/ou un sel de calcium.

Préférentiellement le sel d'acide organique biologique soluble en milieu aqueux utilisé contiendra au moins un sel qui soit un sel de magnésium.

Préférentiellement le sel d'acide organique biologique soluble en milieu aqueux utilisé contiendra au moins un sel qui soit un sel de calcium.

Dans un mode particulier de l'invention la composition selon l'invention comprendra une association de sel de magnésium et de sel de calcium.

Préférentiellement la composition selon l'invention comprendra une association allant de 1/2 de lactate de magnésium et 1/2 de lactate de calcium à une association de 1/4 de lactate de magnésium et de 3/4 de lactate de calcium soit un rapport de 1 pour 1 à 1 pour 3 en poids.

Encore préférentiellement la composition selon l'invention comprendra une association de 2/3 de lactate de magnésium et de 1/3 de lactate de calcium, soit un rapport de 2 pour 1 en poids.

De préférence l'extrait d'algue bleue présent dans la composition représentera entre 0,001 % et 0,1 % en poids du poids total de la composition.

Selon un mode de réalisation avantageux de l'invention, le ou les sel(s) d'acide alpha hydroxylé présent(s) dans la composition représente(nt), ensemble ou chacun considéré séparément, entre 0,001 % et 0,01 % en poids du poids total de la composition.

Selon un mode de réalisation avantageux de l'invention, la composition comprend de 0,001 à 0,01 % d'un sel de magnésium d'acide alpha hydroxylé, (préférentiellement le lactate de magnésium) et de 0,001 à 0,01 % d'un sel de calcium d'acide alpha hydroxylé, (préférentiellement le lactate de calcium).

Dans un mode de réalisation préféré la composition selon l'invention se présentera préférentiellement sous la forme d'un produit de soin et/ou de maquillage de la peau du corps ou du visage, des lèvres, des cils, des sourcils, des cheveux, du cuir cheveu, des ongles ou des muqueuses; d'un produit solaire ou autobronzant; d'un produit capillaire notamment de coloration, de conditionnement et/ou de soin des cheveux.

Préférentiellement la composition selon l'invention comprendra au moins une phase liquide, qui peut comprendre au moins un composé choisi parmi les huiles et/ou solvants d'origine minérale, animale, végétale ou synthétique, carbonés, hydrocarbonés, fluorés et/ou siliconés, sérum, volatils ou non volatils, seuls ou en mélange.

Dans un mode de réalisation préféré la composition selon l'invention sera utilisée dans la prévention de tous les signes liés au vieillissement de l'organisme notamment ceux affectant la peau et/ou les annexes cutanées et/ou les muqueuses.

Préférentiellement la composition selon l'invention sera utilisée dans la prévention des signes de vieillissement cutané.

De préférence la composition selon l'invention est utilisée pour favoriser la production de VEGF (facteur de croissance de l'endothélium vasculaire) pour son utilisation à la revascularisation du derme et l'amélioration du flux sanguin dans les vaisseaux.

Préférentiellement la composition selon l'invention est utilisée pour favoriser la production naturelle de fibres d'élastine.

De préférence la composition selon l'invention est utilisé pour favoriser la production naturelle de collagène.

Préférentiellement la composition selon l'invention est utilisé pour favoriser la production naturelle d'acide hyaluronique.

De préférence la composition selon l'invention est utilisé pour ses propriétés anti-oxydantes.

Préférentiellement la composition selon l'invention sera utilisée dans la prévention et la réparation des affections de la muqueuse buccale.

La présente invention à également pour objet un procédé de préparation selon l'invention comportant les étapes suivantes :

1-Obtention de la biomasse d'algue bleue :

- La biomasse est récoltée après une durée de culture de 10 à 20 jours dans des conditions de température et de luminosité adéquates,

2-Obtention de l'extrait d'algue bleue:

- La biomasse est lavée puis broyée finement. La fraction aqueuse est séparée des débris cellulaires par centrifugation ou filtration,

3-La solution ainsi obtenue est additionnée :

- d'un sel d'acide alpha hydroxylé choisi parmi les sels de magnésium et de calcium et leurs mélanges
- de maltodextrine servant de support

4-Le pH des échantillons est ramené à pH=6,5 en utilisant NaOH (0,1N),

5-Le produit sec est obtenu par élimination de l'eau par une technique appropriée, qui pourra par exemple être: un procédé de lyophilisation, d'atomisation ou de séchage sous étuve ventilée basse température.

Détermination de l'activité anti-oxydante et des concentrations misent en oeuvre:

Afin de déterminer si le lactate de $Mg^{2+}$ (et/ou le lactate de $Ca^{2+}$)présentai(en)t une activité spécifique (et plus particulièrement une activité anti-oxydante, «SOD-like»), plusieurs solutions de sels de lactate (lactate de $Na^+$ ; lactate de $Ca^{2+}$ et lactate de $Mg^{2+}$), à des concentrations différentes, ont été réalisées.

Pour chaque concentration et pour chaque sel, le pH ainsi que l'activité « SOD-like » ont été déterminés.

Des solutions mères (4,0mg/ml) de sels de lactate ont été réalisées à partir des produits suivants :

- lactate de Magnésium - L (FLUKA, p. m.=202,45g/mole)
- lactate de Calcium - L (SIGMA, p. m.=109,1g/mole)
- lactate de Sodium - D/L (SIGMA, p. m.=101,2g/mole)

Chaque concentration a été réalisée par dilutions successives avec de l'eau distillée de pH=6,2.

Les mesures d'activité «SOD-like» sont effectuées à l'aide d'un kit de mesure «Kit SOD525» commercialisé par la société OXIS INTERNATIONAL - BIOXYTECH.

La demanderesse a réalisé chaque mesure de l'activité anti-oxydante en triplicatas.

Tableau 1

| Solutions brutes | Lactate de $Mg^{2+}$ USOD/ml | Lactate de $Ca^{2+}$ USOD/ml | Lactate de $Na^+$ USOD/ml |
|---|---|---|---|
| 0,167mg/ml | 0,14 | 0 | 0 |
| 0,333mg/ml | 0,09 | 0 | 0 |
| 0,667mg/ml | 0,09 | 0 | 0 |
| 1,333mg/ml | 0,69 | 0,21 | 0 |
| 2,0mg/ml | 1,27 | 0,38 | 0,18 |
| 2,66mg/ml | 1,74 | 0,61 | 0,23 |
| 4,0mg/ml | 2,52 | 1,11 | 0,32 |

Les résultats obtenus montrent que le lactate de $Mg^{2+}$ présente une activité «SOD-like» significative à partir d'une concentration de 3mg/1,5ml (soit 2mg/ml). A cette concentration, une activité de 1,27 USOD/ml est mesurée.

Pour des concentrations supérieures à 2mg/ml, l'activité «SOD-like» mesurée augmente à 1,74USOD/ml et 2,52USOD/ml pour des concentrations respectives de 2,66mg/ml et 4mg/ml.

En dessous de cette concentration (0,167mg/ml ; 0,333mg/ml ; 0,667mg/ml ; 1,333mg/ml), l'activité «SOD-like» mesurée n'est pas significative (respectivement 0,14USOD/ml ; 0,09USOD/ml ; 0,09USOD/ml et 0,69USOD/ml) compte tenu du seuil de détection de la méthode utilisée.

En ce qui concerne le lactate de $Ca^{2+}$, les résultats obtenus montrent que ce sel présente une activité «SOD-like» significative (1,11USOD/ml) pour une concentration de 4,0mg/ml.

En dessous de cette concentration (0,167mg/ml ; 0,333mg/ml ; 0,667mg/ml ; 1,333mg/ml ; 2,0mg/ml et 2,66mg/ml), l'activité «SOD-like» mesurée n'est pas significative (respectivement 0USOD/ml ; 0USOD/ml et 0USOD/ml ; 0,21USOD/ml ; 0,38USOD/ml et 0,61USOD/ml).

Enfin, en ce qui concerne le lactate de $Na^+$, les résultats obtenus montrent que ce sel ne présente aucune activité «SOD-like» significative quelle que soit la concentration (0USOD/ml ; 0USOD/ml ; 0USOD/ml ; 0,21USOD/ml ; 0,38USOD/ml et 0,61USOD/ml pour des concentrations respectives de 0,167mg/ml ; 0,333mg/ml ; 0,667mg/ml ; 1,333mg/ml et 2,0mg/ml).

En ce qui concerne le lactate de $Mg^{2+}$, les résultats montrent que la dilution avec de l'eau distillée (pH=6,2) provoque une acidification progressive des solutions (de pH= 8,21 à pH=6,44).

Pour le lactate de $Ca^{2+}$, les résultats obtenus montrent que la dilution avec de l'eau distillée (pH=6,2) provoque également une acidification progressive des solutions (de pH=6,86 à pH=6,2).

Enfin, les résultats obtenus avec le lactate de $Na^+$ montrent que la dilution avec de l'eau distillée (pH=6,2) provoque une alcalinisation progressive des solutions (de pH=5, 27 à pH=5,58).

Tableau 2

| Solutions brutes | Lactate de $Mg^{2+}$ pH | Lactate de $Ca^{2+}$ pH | Lactate de $Na^+$ pH |
|---|---|---|---|
| 0,167mg/ml | 6,44 | 6,20 | 5,58 |
| 0,333mg/ml | 6,64 | 6,33 | 5,54 |
| 0,667mg/ml | 6,88 | 6,44 | 5,49 |
| 1,333mg/ml | 7,05 | 6,64 | 5,47 |
| 2,0mg/ml | 7,44 | 6,68 | 5,46 |
| 2,66mg/ml | 7,53 | 6,74 | 5,37 |
| 4,0mg/ml | 8,21 | 6,86 | 5,27 |

En comparant les résultats obtenus lors de la mesure de l'activité «SOD-like» et lors de la mesure du pH des solutions testées *(cf : tableau 1 et tableau 2)*, la demanderesse a pu établir que :

- Le lactate de $Mg^{2+}$ semble être le plus actif des 3 sels testés puisqu'il présente une activité significative (1,27USOD/ml) pour une concentration de 3mg/ml, alors qu'à cette même concentration aucun des autres sels (lactate de $Ca^{2+}$ et lactate de $Na^+$ ne présentent une activité «SOD-like» équivalente (0,38USOD/ml et 0,18USOD/ml).
- Les activités «SOD-like» sont pratiquement toutes significatives (>1) pour des valeurs de pH proches et/ou supérieures à pH=7,0. En effet, en ce qui concerne le lactate de $Mg^{2+}$, les valeurs significatives de l'activité , «SOD-like» (1,27USOD/ml ; 1,74USOD/ml et 2,52USOD/ml) correspondent à des valeurs respectives de pH de pH=7,44 ; pH=7,53 et pH=8,21.

En ce qui concerne le lactate de Ca2+, une seule valeur significative de l'activité «SOD-like» (1,11USOD/ml) correspond à une valeur de pH de pH=6,86. Enfin, en ce qui concerne le lactate de Na+, aucune valeur significative de l'activité «SOD-like» n'est observée étant donné le caractère très acide de ce sel.

La demanderesse à donc mis en évidence que le lactate de $Mg^{2+}$ présente la plus forte activité «SOD-like» parmi les 3 sels de lactate testés. Cependant, compte tenu du fait qu' il semble exister une corrélation étroite entre activité «SOD-like» et pH, des mesures complémentaires de l'activité «SOD-like» des trois sels, pour des valeurs de pH identiques ont été effectuées.

Le pH des échantillons à été ramené à pH=6,5 en utilisant NaOH (0,1N)

Tableau 3

| Solutions brutes | Lactate de Mg$^{2+}$ pH=6,0 (USOD/ml) | Lactate de Mg$^{2+}$ pH=7,0 (USOD/ml) | Lactate de Mg$^{2+}$ pH=7,4 (USOD/ml) | Lactate de Mg$^{2+}$ pH=8,0 (USOD/ml) |
|---|---|---|---|---|
| 0,167mg/ml | 0 | 0 | 0 | 0 |
| 0,333mg/ml | 0 | 0 | 0 | 0 |
| 0,667mg/ml | 0,21 | 0,32 | 0,25 | 0 |
| 1,333mg/ml | 0,6 | 0,61 | 0,48 | 0,41 |
| 2,0mg/ml | 2,62 | 2,64 | 1,7 | 1,41 |
| 2,66mg/ml | 3,95 | 3,94 | 3,64 | 3,63 |
| 4,0mg/ml | 7,21 | 7,32 | 6,87 | 6,23 |

La demanderesse a donc décidé d'effectuer des mesures d'activités «SOD-like» de ces deux sels, aux mêmes concentrations, et pour des valeurs de pH identiques (pH=6,0; pH=7,0 ; pH=7,4 et pH= 8,0).

Les solutions mères (4,0mg/ml) de sels de lactate de magnésium et de calcium sont les mêmes que décrites précédemment.

Les résultats obtenus (tableau 3) semblent démontrer que pour une même concentration en sel de lactate de Mg$^{2+}$, les activités «SOD-like» mesurées varient linéairement en fonction du pH. Ce résultat confirme donc l'influence du pH sur L'activité «SOD-like».

En outre, les résultats montrent que le sel de lactate de Mg$^{2+}$ (quelles que soient les concentrations) présente une activité «SOD-like» optimale pour une valeur de pH=7,0. L'activité «SOD-like» du sel de lactate de Mg$^{2+}$ correspondant au pH biologique (pH=7,4) est légèrement inférieure à l'activité «SOD-like» optimale.

Enfin, les résultats confirment les résultats obtenus précédemment quant au seuil de concentration minimal 2,5mg/1,5ml (soit 1,67mg/ml) nécessaire en sel de lactate de Mg$^{2+}$, pour obtenir une activité «SOD-like» significative.

La demanderesse a décidé d'effectuer les mêmes mesures à pH=6 ; pH=7,0 et pH=7,5 pour le lactate de Ca$^{2+}$.

Tableau 4

| Solutions brutes | Lactate de Ca$^{2+}$ pH=6,0 (USOD/ml) | Lactate de Ca$^{2+}$ pH=7,0 (USOD/ml) | Lactate de Ca$^{2+}$ pH=7,5 (USOD/ml) |
|---|---|---|---|
| 0,167mg/ml | 0 | 0 | 0 |
| 0,333mg/ml | 0 | 0 | 0 |
| 0,667mg/ml | 0 | 0 | 0 |
| 1,333mg/ml | 0,72 | 0,65 | 0,57 |
| 2,0mg/ml | 0,93 | 0,93 | 0,8 |
| 2,66mg/ml | 2,69 | 2,68 | 2,12 |
| 4,0mg/ml | 3,5 | 2,93 | 2,57 |

Les résultats obtenus (tableau 4) démontrent que pour une même concentration en sel de lactate de Ca$^{2+}$, les activités «SOD-like» mesurées varient linéairement en fonction du pH. Ce résultat confirme donc l'influence du pH sur l'activité «SOD-like» du sel de lactate de Ca$^{2+}$, comme pour le sel de lactate de Mg$^{2+}$.

En outre, les résultats montrent que le sel de lactate de Ca$^{2+}$ (quelles que soient les concentrations) présente une activité «SOD-like» optimale pour une valeur de pH égale à pH=6,0.

Les résultats obtenus semblent d'une montrer que le pH joue un rôle dans l'activité «SOD-like» des sels de lactate de Mg$^{2+}$ et de lactate de Ca$^{2+}$. Pour ces deux sels, l'activité «SOD-like» maximale semble être déterminée pour une valeur de pH compris entre pH=6,0 et pH=7,0.

La Demanderesse a décidé de réaliser des mesures d'activités «SOD-like» sur des mélanges des deux sels de lactate de Mg$^{2+}$ et lactate de Ca$^{2+}$, afin de mettre en évidence une éventuelle synergie. Les mesures ont été réalisées pour une valeur de pH=6,5 qui correspond à une activité «SOD-like» maximale et qui constitue la valeur généralement utilisée dans les applications en dermo-cosmétique.

Les solutions mères (4,0mg/ml) de sels de lactate de magnésium et de calcium sont les mêmes que décrites précédemment.

Tableau 5

| Solutions brutes Lactate de $Mg^{2+}/Ca^{2+}$ (Proportion 3/1) | Lactate de $Mg^{2+}/Ca^{2+}$ pH=6,5 (USOD/ml) | Lactate de $Mg^{2+}$ +Lactate de $Ca^{2+}$ pH=6,5 (USOD/ml) |
|---|---|---|
| 0,25mg/0,083mg//ml | 0,08 | 0 |
| 0,5mg/0,167mg//ml | 0,3 | 0,1 |
| 1,0mg/0,33mg//ml | 1,08 | 0,4 |
| 2,0mg/0,667mg//ml | 3,53 | 2,62 |
| 4,0mg/1,33mg//ml | 7,57 | 7,93 |

Tableau 6

| Solutions brutes Lactate de $Mg^{2+}/Ca^{2+}$ (Proportion 1/1) | Lactate de $Mg^{2+}/Ca^{2+}$ pH=6,5 (USOD/ml) | Lactate de $Mg^{2+}$ +Lactate de $Ca^{2+}$ pH=6,5 (USOD/ml) |
|---|---|---|
| 0,167mg/0,167mg//ml | 0 | 0 |
| 0,33mg/0,33mg//ml | 0 | 0 |
| 0,667mg/0,667mg//ml | 0 | 0 |
| 1,0mg/1,0mg//ml | 1,48 | 0,7 |
| 2,0mg/2,0mg//ml | 4,68 | 3,55 |

Les résultats obtenus lors de cette expérience (tableau 5 et tableau 6) montrent que les activités «SOD-like» mesurées sur les mélanges de sels de lactate de $Mg^{2+}/Ca^{2+}$ (1ères colonnes des tableaux 5 et 6) sont globalement toutes supérieures à la somme des activités des deux sels pris séparément (2èmes colonnes des tableaux 5 et 6).

En outre, en comparant les résultats obtenus sur les différents mélanges, il apparaît que la proportion de type 3/1 ($Mg^{2+}/Ca^{2+}$) (tableau 5) soit plus efficace que la proportion de type 1/1 ($Mg^{2+}/Ca^{2+}$) (tableau 6).

En effet, même s'il existe une synergie pour les proportions de type 1/1, les activités obtenues dans la proportion 3/1 sont supérieures à celles obtenues dans les proportions 1/1 pour des concentrations totales équivalentes (ex: 0,3USOD/ml et 1,0SUSOD/ml contre 0USOD/ml et 0USOD/ml pour des concentrations totales respectives de 0,667mg/ml et 1,333mg/ml en sels). Ce résultat est en accord avec les expériences précédentes qui montraient que le sel de lactate de $Mg^{2+}$ présentait une activité «SOD-like» supérieure à celle du sel de lactate de $Ca^{2+}$.

De plus, il est important de noter qu'une activité de 1,08USOD/ml est obtenue pour le mélange 1mg lactate de $Mg^{2+}/0.33mg$ lactate $Ca^{2+}//ml$.

Mesure l'activité «SOD-like» des lactates associés à des algues bleues :

Les lots N°007 et PF (1,2,3,4 et 5) sont des lots préparés selon le cahier des charges des laboratoires PIERRE FABRE à partir de lots de *phormidium sp.*.

Obtention de la biomasse :

La biomasse est récoltée après une durée de culture de 10 à 20 jours dans des conditions de température et de luminosité adéquates.

Obtention de l'extrait d'algues bleues:

La biomasse est lavée puis broyée finement. La fraction aqueuse est séparée des débris cellulaires par centrifugation ou filtration.

La solution ainsi obtenue est additionnée :

- d'acide alpha hydroxylé ou un de ses sels
- de maltodextrine servant de support

Le pH des échantillons à été ramené à pH=6,5 en utilisant NaOH (0,1N)

Le produit sec est obtenu par élimination de l'eau par une technique appropriée, qui pourra par exemple être: un procédé de lyophilisation, d'atomisation ou de séchage sous étuve ventilée basse température.

L'ajout en sels à été réalisé par ajout des sels de lactate de magnésium et de calcium précédemment utilisés.

L'activité «SOD-like» des échantillons a été déterminée avec le même kit et selon la même méthode que précédemment. Résultat de l'activité «SOD-like» pour des associations contenant l'extrait de Phormidium et le mélange de sels de lactates.

Tableau 7

| Identification des lots | Concentration en mélange actif 2/3 lactate de $Mg^{2+}$ et 1/3 lactate de $Ca^{2+}$ | Activité "SOD-like" |
|---|---|---|
| PF1 | 0,45% | 4,5U |
| PF2 | 0,30% | 4U |
| PF3 | 0,23% | 2,1U |
| PF4 | 0,15% | 1U |
| PF5 | 0% | 0,6U |

Les résultats présentés dans le tableau 7 représentent les différentes activités «SOD-like» mesurées pour le mélange de sels de lactate de $Mg^{2+}/Ca^{2+}$ en proportion 2/3 de lactate de $Mg^{2+}$ et 1/3 de lactate de $Ca^{2+}$.

On constate que la concentration optimale est obtenue pour une concentration du mélange actif de 0.30% (lot JCB2.

En outre, en comparant ces résultats avec ceux obtenus précédemment sur les différents mélanges, il apparaît que la proportion de type 2/1 ($Mg^{2+}/Ca^{2+}$) (tableau 7) est plus efficace que les proportions de type 1/1 et 3/1($Mg^{2+}/Ca^{2+}$).

Tableau 8

| Echantillons | Activité "SOD-like" |
|---|---|
| Lot 007 (Ph=6,2: lot initial) | 0,81U |
| Lot 007 (pH=6,5) | 1,07U |
| Lot 007 (pH=6,5 + 0,5mg/ml lactate de $Mg^{2+}$) | 1,21U |
| Lot 007 (pH=6,5 +1,0mg/ml lactate de $Mg^{2+}$) | 1,93U |
| Lot 007 (pH=6,5 +1,5mg/ml lactate de $Mg^{2+}$) | 3,57U |
| Lot 007 (pH=6,5 +2,0mg/ml lactate de $Mg^{2+}$) | 4,29U |
| Lot 007 (pH=6,5 +5,0mg/ml lactate de $Mg^{2+}$) | 4,4U |

Les résultats obtenus lors de la surcharge du lot 007 au lactate de $Mg^{2+}$ (Tableau 8) semblent montrer que lorsque le pH du lot 007 (activité, «SOD-like» initiale: 0,81USOD525/ml) est ramené à pH=6,5, l'activité augmente jusqu'à une valeur significative de 1,07 USOD525/ ml.

En outre, les résultats obtenus semblent démontrer que l'ajout de lactate de $Mg^{2+}$ dans l'extrait initial, augmente significativement son activité «SOD-like». En effet, en ajoutant respectivement 0,5mg/ ml de lactate de $Mg^{2+}$ ; 1,0mg/ml de lactate de $Mg^{2+}$, 1,5mg/ml de lactate de $Mg^{2+}$, 2,0mg/ml de lactate de $Mg^{2+}$ et 5,0mg/ml de lactate de $Mg^{2+}$, l'activité «SOD-like» augmente respectivement à 1,21USOD525/ml 1,93USOD525/ml, 3,57USOD525/ml, 4,29USOD52/ml et 4,4USOD52S/ml.

Enfin, il est important de noter que l'activité «SOD-like» de l'extrait semble atteindre un maximum (4,29USODs25/ml) à partir d'une concentration de 2,0mg/ml de lactate de $Mg^{2+}$.

Tableau 9

| Echantillons | Activité "SOD-like" |
|---|---|
| Lot 007 (Ph=6,2: lot initial) | 0,81U |
| Lot 007 (pH=6,5) | 1,07U |
| Lot 007 (pH=6,5 + 0,5mg/ml lactate de $Ca^{2+}$) | 1,11U |
| Lot 007 (pH=6,5 +1,0mg/ml lactate de $Ca^{2+}$) | 1,52U |
| Lot 007 (pH=6,5 +1,5mg/ml lactate de $Ca^{2+}$) | 1,77U |
| Lot 007 (pH=6,5 +2,0mg/ml lactate de $Ca^{2+}$) | 2,14U |
| Lot 007 (pH=6,5 +5,0mg/ml lactate de $Ca^{2+}$) | 3,83U |

Les résultats obtenus (tableau 9) montrent que l'ajout de lactate de Ca2+ dans l'extrait initial, augmente significativement son activité «SOD-like».

En effet, en ajoutant respectivement 0,5mg/ml de lactate de $Ca^{2+}$; 1,0mg/ml de lactate de $Ca^{2+}$, 1,5mg/ml de lactate de $Ca^{2+}$, 2,0mg/ml de lactate de $Ca^{2+}$ et 5,0mg/ml de lactate de Ca2+, l'activité «SOD-like» augmente respectivement à 1,11USOD525/ml; 1,52USOD525/ml, 1,77USOD525/ml, 2,14USOD525/ml et 3,83USOD525/ml.

Enfin, au cours de l'expérience, il ne semble pas que l'activité «SOD-like» de l'extrait ait atteint un maximum (3,83USOOS2S/ ml) puisqu'elle augmente régulièrement au fur et à mesure que l'on ajoute du lactate de $Ca^{2+}$.

Les résultats obtenus lors de l'expérience de surcharge du lot 007 au lactate de $Mg^{2+}$ semblent montrer qu'il est intéressant de ramener le pH de l'extrait 007 à pH=6,5 afin d'augmenter son activité «SOD-like».

En outre, l'ajout de lactate de $Mg^{2+}$ semble augmenter significativement l'activité «SOD-like» de l'extrait (jusqu'à 4,29USOD525/ml pour 2mg/ml). Il est important de noter que dans le cas du lactate de $Mg^{2+}$, cette augmentation de l'activité «SOD-like» semble exponentielle et semble atteindre un niveau maximal d'activité à partir de 2mg/ml.

Enfin, en comparaison avec les résultats présentés précédemment, l'ajout de lactate de $Mg^{2+}$, à raison de 2mg/ ml, permet une augmentation de l'activité de l'extrait de 3,22USOD525/ml (en tenant compte de son activité initiale soit 1,07USOD525/ml), alors que l'activité obtenue avec 2mg/ml de lactate de $Mg^{2+}$ seul, était environ de 2,6USOD525/ml.

Description des figures :

Figure 1 : Représentation de la migration cellulaire des kératinocytes (en IF) après migration pendant 24h de Kératinocytes HaCaT.

Figure 2 : Représentation de l'expression de VEGF sécrété par les kératinocytes HaCaT après migration pendant 24h.

Figure 3 : Représentation de l'expression de l'acide hyaluronique sécrété par les kératinocytes HaCaT après migration pendant 24h par dosage colorimétrique.

Figure 4 : Révélation avec immunomarquage de l'effet de « l'association » sur l'expression du récepteur CD44 sur des kératinocytes HaCaT incubés pendant 24h. Le CD 44 est visualisé par une fluorescence verte et le noyau des cellules par une fluorescence bleu (coloration DAPI).

Figure 5 : Représentation de l'activité anti-radicalaire de différents extraits d'algues bleues incubés pendant 24 heures vis à vis de la lipoperoxydation induite par un stress chimique de 1 heure sur fibroblastes L929.

Figure 6 : Représentation de l'expression de l'élastine et du collagène I (ARNm) sur fibroblastes rendus sénescents par un stress oxydatif ($H_2O_2$) après 72 heures. « L'association » est pré-incubée pendant 16 heures.

Figure 7 : Représentation de l'expression des métalloprotéinases matricielles (MMP-1, MMP-3 et MMP-12) (ARNm) sur fibroblastes rendus sénescents par réplication « L'association » est pré-incubée pendant 72 heures.

Les exemples suivants illustrent l'invention sans en limiter la portée.

Les tests ont été réalisés avec une association, ci-après dénommée « l'association », composée d'un extrait d'algues bleues de type *Phormidium sp.* pour 1/3 en poids et d'un mélange de lactate de magnésium et de lactate de calcium (dans un rapport de 2 pour 1) pour 2/3 en poids.

Exemple 1: Etude de l'induction de la migration des kératinocytes

La migration des kératinocytes, constituants principaux de l'épiderme, joue un rôle primordial dans la réparation tissulaire et la cicatrisation. Afin de déterminer les effets de « l'association » sur la migration des kératinocytes, la demanderesse a utilisé comme modèle cellulaire des Kératinocytes HaCaT.

Après migration pendant 24h des Kératinocytes avec des Kit Oris Cell Migration Assay (96 puits), la demanderesse a effectué un dosage mesurant l'intensité de fluorescence (IF).

L'induction de la migration des kératinocytes à été évaluée avec un témoin selon :

- Sels de Lactates seuls 2000µg/ml
- Extrait d'algues bleues seules 110µg/ml
- – « L'association » = 200µg/ml sels lactates + 110µg/ml algues bleues

« L'association » = 200µg/ml sels lactates + 110µg/ml algues bleues

Témoin positif :

- TGF1β1 5ng/ml

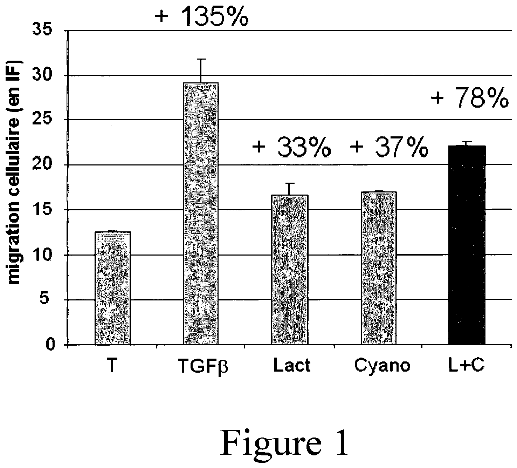

Les résultats de la migration sont représentés à la figure 1.

Figure 1 : Représentation de la migration cellulaire des kératinocytes (en IF) après migration pendant 24h de Kératinocytes HaCaT.

On distingue très nettement l'induction de la migration des kératinocytes en comparaison avec TGF beta 5ng/ml. Les extraits d'algues bleues seuls ou les sels de lactate seuls induisent une migration modérée (respectivement +33% et +37%) visible sur la figure 1.

« L'association » révèle une véritable synergie avec une induction de la migration des kératinocytes à +78%.

Exemple 2: Production de facteurs sécrétés par les kératinocytes : VEGF

Le VEGF (pour Vascular Endothelial Growth Factor ou Facteur de croissance endothélial vasculaire) a plusieurs rôles biologiques importants. Par exemple, il limite la prolifération des cellules endothéliales, active la formation de nouveaux vaisseaux sanguins (angiogenèse) et accroît la perméabilité vasculaire. Ce faisant, le VEGF sécrété par les kératinocytes contribue au maintien d'une peau saine par apport d'éléments nutritifs aux cellules et favorise la cicatrisation.

L'angiogenèse est un mécanisme de néovascularisation prenant naissance à partir d'un réseau capillaire préexistant. Elle est particulièrement importante et indispensable au cours de nombreux processus physiologiques tels que le développement embryonnaire, l'implantation du placenta ou la réparation tissulaire.

Afin d'effectuer le dosage de la libération de VEGF par les kératinocytes en culture, la demanderesse à dosé, par technique ELISA, la quantité du Facteur de croissance endothélial vasculaire sécrété présente dans les surnageants des expériences de migration sur kératinocytes HaCaT comme décrit dans l'exemple 1.

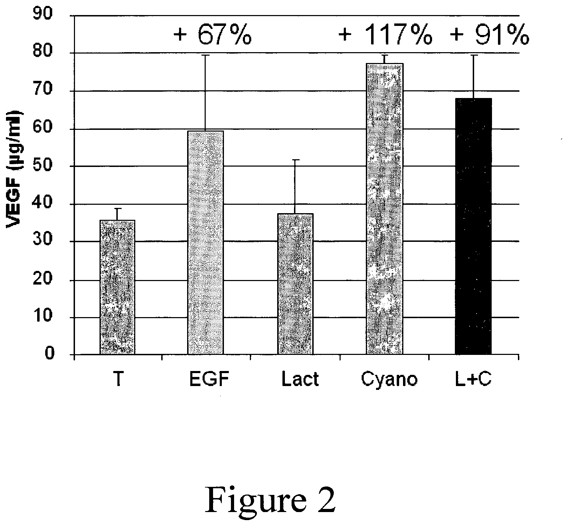

Les résultats de ces mesures sont visibles dans la figure 2.

Figure 2 : Représentation de l'expression de VEGF sécrété par les kératinocytes HaCaT après migration pendant 24h.

Il apparaît que l'induction de la sécrétion de VEGF est principalement le résultat des algues bleues. En effet, la quantité de VEGF mesurée avec l'extrait d'algues bleues seuls (+117%) et « l'association » (+91%) est considérablement plus élevé qu'avec le témoin positif ayant donné le meilleur résultat EGF (67%).

Les algues bleues grâce à l'induction de la sécrétion de VEGF favorisent donc la revascularisation des tissus et la régénération de ceux-ci. La composition selon l'invention contribue ainsi au maintien d'une peau saine, favorise la cicatrisation et aide au comblement des rides.

Exemple 3: Production d'acide hyaluronique (AH) et de son récepteur le CD44 sur les kératinocytes

L'antigène CD44 représente une famille de glycoprotéines transmembranaires de type I. La fonction de CD44 comme récepteur de l'acide hyaluronique, un composant crucial de la matrice extracellulaire, est bien connu. L'acide hyaluronique est un glycosaminoglycane, réparti largement parmi les tissus conjonctifs, épithéliaux et nerveux mais que l'on retrouve dans tous les tissus. C'est l'un des principaux composants de la matrice extracellulaire. Il contribue de façon significative à la prolifération et à la migration des cellules.

Afin d'effectuer le dosage du relargage de l'acide hyaluronique par les kératinocytes en culture, la demanderesse a dosé la quantité d'AH sécrétée présente dans les surnageants dans une expérience de migration sur kératinocytes HaCaT comme décrit dans l'exemple 1.

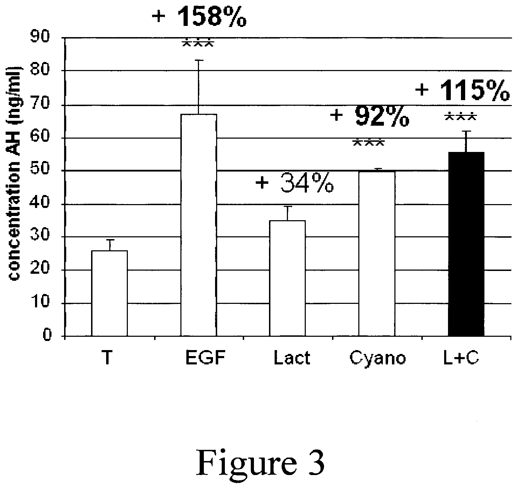

L'acide hyaluronique est ensuite mesuré par ELISA compétitif analysé en colorimétrie et exprimé en ng/ml en comparaison avec un témoin non stimulé et un témoin positif (EGF). Les résultats de ces mesures sont visibles dans la figure 3.

Figure 3 : Représentation de l'expression de l'acide hyaluronique sécrété par les kératinocytes HaCaT après migration pendant 24h par dosage colorimétrique.

Il apparaît que l'induction du relargage d'acide hyaluronique est plus importante avec les extraits d'algues bleues seuls (92%) qu'avec les sels de lactates seuls (34%).

L'association algues bleues/Sels de lactates $Ca^{2+}/Mg^{2+}$ selon l'invention présente quant à elle un effet additif par rapport aux extraits seuls avec 115% d'augmentation de l'acide hyaluronique mesuré dans le surnageant par rapport au témoin.

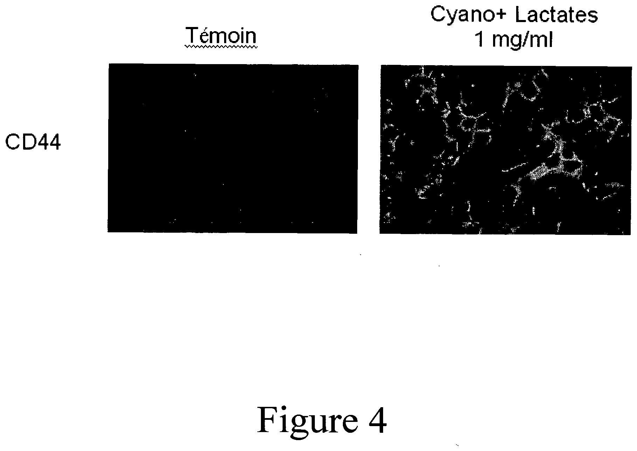

Afin d'évaluer l'expression du récepteur membranaire CD44 par les kératinocytes en culture, la demanderesse a effectué un immunomarquage à l'aide d'un anticorps anti-CD44 après 24 heures d'incubation en présence de « l'association » en comparaison d'un témoin neutre (figure 4).

Figure 4 : Révélation avec immunomarquage de l'effet de « l'association » sur l'expression du récepteur CD44 sur des kératinocytes HaCaT incubés pendant 24h. Le CD 44 est visualisé par une fluorescence verte et le noyau des cellules par une fluorescence bleu (coloration DAPI).

Ce résultat montre que « l'association » induit une expression du récepteur CD44 supérieure à l'expression basale.

Les effets de « l'association » sur l'induction de l'expression de l'acide hyaluronique et de son récepteur CD44, sont intéressants pour favoriser le comblement des rides dans une indication anti-âge, l'hydratation et la régénération tissulaire.

Exemple 4: Activité anti-oxydante sur fibroblastes

La membrane plasmique constitue la principale et la première cible des radicaux libres oxygénés (RLO). Elle est le site d'une peroxydation accrue de par sa richesse en lipides. Pour mesurer la peroxydation des lipides membranaires, la Demanderesse a réalisé un dosage *in vitro* des complexes entre les produits d'oxydations lipidiques et l'acide thiobarbiturique. Ces complexes sont appelés TBARS (pour Thiobarbituric Acid Reactive Substances) et donnent son nom au test: Test des TBARS.

Afin de reproduire les effets d'un stress oxydatif chimique, la Demanderesse a traité, pendant 1 heure, une lignée de fibroblastes, L929, par un complexe composé de peroxyde d'hydrogène ($H_2O_2$) et de fer ($Fe^{2+}/Fe^{3+}$) reconstituant ainsi la réaction de Fenton, source de RLO et plus particulièrement de radical hydroxyle (OH°) : $H_2O_2 + Fe^{2+} \rightarrow$ OH° + OH⁻ + $Fe^{3+}$.

Ce modèle a permis d'évaluer l'activité antiradicalaire de différents extraits d'algues bleues, et d'analyser leur pouvoir protecteur vis-à-vis de la lipoperoxydation induite par un stress chimique.

La demanderesse a déterminé l'activité anti-oxydante des différentes associations selon l'invention après 24 heures d'incubation en mesurant la protection des différentes compositions contre la peroxydation lipidique en comparaison avec la protection apportée par la vitamine E en tant que témoin positif.

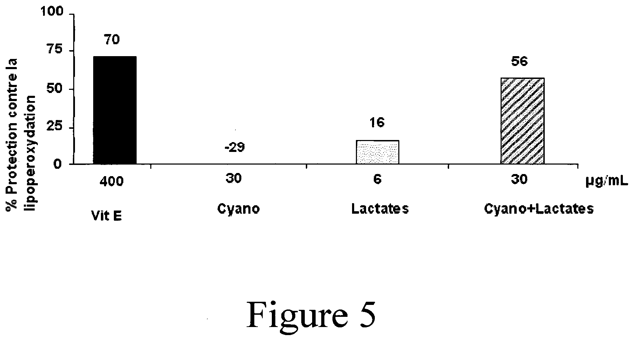

Les résultats sont présentés sur la figure 5.

Figure 5 : Représentation de l'activité anti-radicalaire de différents extraits d' algues bleues incubés pendant 24 heures vis à vis de la lipopéroxydation induite par un stress chimique de 1 heure sur fibroblastes L929.

La demanderesse a pu montrer que l'extrait d'algues bleues seul n'avait pas d'activité mesurable alors que les sels de lactates seuls présentaient une faible activité (16%).

« L'association » révèle une véritable synergie avec une un pourcentage de protection de 56% proche de celui obtenu avec la vitamine E (70%).

L'association algues bleues/Sels de lactates selon l'invention présente donc une bonne activité anti-oxydante.

Exemple 6: Synthèse d'élastine et de collagène I sur fibroblastes

Le collagène de type I est un collagène de type fibrillaire majeur. Il est présent dans la peau, les os, les tendons, la cornée et les organes internes. Le collagène I représente 90% du collagène que contient le corps humain. Il est constitué de trois chaînes polypeptidiques enroulées en hélice dans une partie de la molécule.

L'élastine est une protéine fibreuse riche en proline. Les fibres élastiques qui donnent à la peau son élasticité sont constituées d'un noyau d'élastine entouré d'un manchon de microfibrilles.

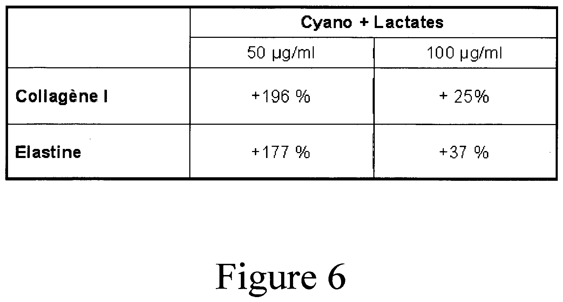

Figure 6 : Représentation de l'expression de l'élastine et du collagène I (ARNm) sur fibroblastes rendus sénescents par un stress oxydatif ($H_2O_2$) après 72 heures. « L'association » est pré-incubée pendant 16 heures.

Les effets de « l'association » sur l'induction de la synthèse d'élastine et de collagène I sur les fibroblastes, permettent donc de favoriser le comblement des rides dans un indication anti-âge.

Exemple 7: Inhibition de MMP-1, MMP-3, MMP-12

Les métalloprotéinases matricielles ou MMP (de l'anglais *Matrix metalloproteinase*) sont des enzymes qui dégradent les constituants de la matrice extracellulaire des tissus conjonctifs, notamment de la peau.

Ces métalloprotéinases sont des enzymes à zinc ayant une activité endopeptidasique, c'est-à-dire qu'elles hydrolysent les protéines. La majorité des métalloprotéinases sont sécrétées par les cellules dans l'espace extracellulaire. Il en existe de nombreux types (une trentaine environ) dont :

- La collagénase intersticielle ou MMP1 : synthétisée par les fibroblastes ou les polynucléaires, elle dégrade les collagènes fibrillaires I, II et III.
- La stromélysine ou MMP3 : qui dégrade les constituants de la lame basale et l'élastine.
- La MMP12, principalement exprimé par les macrophages qui joue un rôle dans les phénomènes d'inflammation et de cicatrisation.

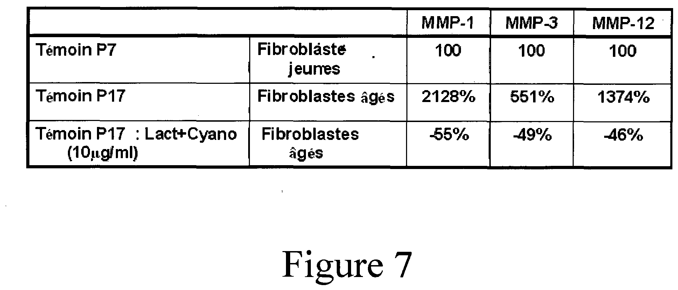

Figure 7 : Représentation de l'expression des métalloprotéinases matricielles (MMP-1, MMP-3 et MMP-12) (ARNm) sur fibroblastes rendus sénescents par réplication « L'association » est pré-incubée pendant 72 heures.

« L'association » révèle une véritable inhibition de la synthèse des métalloprotéinases matricielles (MMP-1, MMP-3 et MMP-12)dans les fibroblastes sénescents. En ralentissant la dégradation des constituants de la matrice extracellulaire l'association selon l'invention montre sa capacité à lutter contre le vieillissement cutané.

Exemple 8: Exemples de compositions cosmétiques selon l'invention

formule 1 :

| | |
|---|---|
| -eau | 40 - 65% |
| -tribehenin PEG-20 esters | 2 - 7% |
| -isodécyl néopentanoate | 2 - 9% |
| -glycérine | 0.5 - 10% |
| -PEG-8 | 0.5 - 8% |
| -dimethicone | 0.5 - 5% |
| -palmitate glycol | 1 - 6% |
| -alcool cétyl | 0.5 - 3% |
| -BHT | 0.01 - 0.0-3% |
| -disodium EDTA | 0.05 - 0.25% |
| -lécithine hydrogénée | 0.25 - 0.8% |
| -conservateur | 0.5 - 3% |
| -parfum | 0.2 - 0.5% |
| -gomme de xanthane | 0.1 - 0.4% |
| avec -lactate de calcium | 0,001 - 0,1% |
| -lactate de magnésium | 0,001 - 0,1% |
| -maltodextrine | 0,1 - 1% |
| -phormidium uncinatum | 0,001 - 0,1% |

formule 2:

| | |
|---|---|
| - eau | 40 - 85% |
| -isodécyl néopentanoate | 2 - 9% |
| -glycérine | 0.5 - 10% |
| -alcool d'arachide | 0.5 - 3% |
| -alcool behenyl | 0.3 - 2% |

(suite)

| | |
|---|---|
| -disodium EDTA | 0.05 - 0.25% |
| -glycéryl stéarate | 0.5 - 4% |
| -lecithine hydrogénée | 0.25 - 0.8% |
| -conservateur | 0.5 - 3% |
| -parfum | 0.2 - 0.5% |
| -mica | 0.001 - 1% |
| -polyméthyl métacrylate | 0.01 - 0.9% |
| -tin oxide | 0.001 - 0.65% |
| -dioxide de titanium | 0.01 - 0.9% |
| -gomme de xanthane | 0.1 - 0.4% |
| avec -lactate de calcium | 0,001 - 0,1% |
| -lactate de magnésium | 0,001 - 0,1% |
| -maltodextrine | 0,1 - 1% |
| -phormidium uncinatum | 0,001 - 0,1% |

De manière non limitative, pourront notamment être utilisés seuls ou en mélanges:

Comme émulsionnants:

tribehenin PEG- 20 esters, lécithine hydrogénée, alcool cétéaryl, cétéaryl glucoside, glyceryl stérate, PEG100 stéarate, potassium cétyl phosphate, sodium stéaroyl glutamate, alcool arachidyl, glucoside arachidyl, alcool cétyl.

Comme émollients:

isodécyl néopentanoate, dicaprylyl carbonate, ppg-15 stéarylether,C12-15alkylbenzoate, isododécane , isohexadecane.

Comme humectants:

glycérine, PEG-8, méthyl gluceth-20, butylène glycol, sorbitol,dipropylène glycol, PEG-4 , PEG-12.

Comme agents de texture:

diméthicone, glycol palmitate, behenyl alcool, glycérol monostéarate, polyméthyl métacrylate, nylon 12.

Comme anti-oxydants:

BHT, tocophérol, tocophéryl acetate.

Comme conservateurs:

disodium EDTA, tetrasodium EDTA, trisodium éthylènediamine diccinate.

Comme gélifiants:

Gomme de xanthane, gomme de gellane, magnesium aluminium silicate,acrylates copolymères, hydroxyéthylcellulose,polymère carboxyvynilique.

Comme colorants :

mica, oxide de tin, dioxide de titanium.

**Revendications**

1. Composition topique **caractérisée en ce qu'**elle contient au moins un extrait d'algue bleue de type *Phormidium sp.* et au moins un sel d'acide alpha hydroxylé soluble en milieu aqueux, choisi parmi les sels de magnésium et de calcium ainsi que leurs mélanges, en association avec au moins une excipient acceptable pour l'application topique.

2. Composition topique selon la revendication 1, **caractérisée en ce que** ledit sel d'acide alpha hydroxylé est un lactate.

3. Composition topique selon l'une des revendications 1 à 2, **caractérisée en ce qu'**elle contient au moins un sel de magnésium.

4. Composition topique selon l'une des revendications 1 à 2, **caractérisée en ce qu'**elle contient au moins un sel de calcium.

5. Composition topique selon l'une des revendications 1 à 2, **caractérisée en ce qu'**elle comprend une association de sel de magnésium et de sel de calcium.

6. Composition topique selon la revendication 5, **caractérisée en ce qu'**elle comprend une association allant de 1/2 de lactate de magnésium et 1/2 de lactate de calcium à une association de 1/4 de lactate de magnésium et de 3/4 de lactate de calcium soit un rapport de 1 pour 1 à 1 pour 3 en poids total de la composition.

7. Composition topique selon la revendication 6, **caractérisée en ce qu'**elle comprend une association de sel de magnésium et de sel de calcium dans une proportion de 2/3 // 1/3 soit un rapport de 2 pour 1 en poids.

8. Composition topique selon l'une quelconque des revendications précédentes, **caractérisée en ce que** l'extrait d'algue bleue présent dans la composition représente entre 0,001% et 0,01% en poids, du poids total de la composition.

9. Composition topique selon l'une quelconque des revendications précédentes, **caractérisée en ce que** le ou les sel(s) d'acide alpha hydroxylé présent(s) dans la composition représente(nt), ensemble ou chacun considéré séparément, entre 0,001% et 0,01% en poids, du poids total de la composition.

10. Composition topique selon l'une des revendications 1 à 9, **caractérisée en ce qu'**elle se présente sous la forme d'un produit de soin et/ou de maquillage de la peau du corps ou du visage, des lèvres, des cils, des sourcils, des cheveux, du cuir chevelu, des ongles ou des muqueuses; d'un produit solaire ou autobronzant; d'un produit capillaire notamment de coloration, de conditionnement et/ou de soin des cheveux.

11. Composition topique selon l'une des revendications 1 à 9, **caractérisée en ce qu'**elle comprend au moins une phase liquide comprenant au moins un composé choisi parmi les huiles et/ou solvants d'origine minérale, animale, végétale ou synthétique, carbonés, hydrocarbonés, fluorés et/ou siliconés, sérum, volatils ou non volatils, seuls ou en mélange.

12. Utilisation cosmétique d'une composition topique selon l'une des revendications 1 à 11, dans la prévention et/ou le traitement de tous les signes liés au vieillissement de l'organisme notamment ceux affectant la peau et/ou les annexes cutanées et/ou les muqueuses.

13. Utilisation cosmétique selon la revendication 12, dans la prévention des signes de vieillissement cutané.

14. Composition topique selon l'une des revendications 1 à 9 pour son utilisation dans la prévention et/ou le traitement des affections de la muqueuse buccale.

15. Procédé de traitement cosmétique des matières kératiniques, notamment de la peau du corps ou du visage, des lèvres, des ongles, des cheveux, des cils et/ou des muqueuses, comprenant l'application sur lesdites matières d'une composition cosmétique telle que définie à l'une des revendications 1 à 9.

16. Procédé de préparation de compositions selon les revendications 1 à 11 **caractérisé en ce qu'**il comporte les étapes suivantes :

1-Obtention de la biomasse d'algue bleue:

- La biomasse est récoltée après une durée de culture de 10 à 20 jours dans des conditions de température et de luminosité adéquates,

2-Obtention de l'extrait d'algue bleue:

- La biomasse est lavée puis broyée finement. La fraction aqueuse est séparée des débris cellulaires par centrifugation ou filtration,

3-La solution ainsi obtenue est additionnée :

- d'un sel d'acide alpha hydroxylé choisi parmi les sels de magnésium, de calcium et leurs mélanges
- de maltodextrine servant de support

4-Le pH des échantillons est ramené à pH=6,5 en utilisant NaOH (0,1N),
5-Le produit sec est obtenu par élimination de l'eau par une technique appropriée, qui pourra par exemple être: un procédé de lyophilisation, d'atomisation ou de séchage sous étuve ventilée basse température.

## Patentansprüche

1. Topische Zusammensetzung, **dadurch gekennzeichnet, dass** sie mindestens einen Blaualgenextrakt vom Typ *Phormidium sp.* und mindestens ein alpha-Hydroxysäuresalz, das in wässrigem Medium löslich ist und aus Magnesium-, und Calcium- sowie deren Gemischen ausgewählt ist, im Verbund mit mindestens einem Hilfsstoff, der zur topischen Anwendung akzeptabel ist, enthält.

2. Topische Zusammensetzung nach Anspruch 1, **dadurch gekennzeichnet, dass** das besagte alpha-Hydroxysäuresalz ein Lactat ist.

3. Topische Zusammensetzung nach einem der Ansprüche 1 bis 2, **dadurch gekennzeichnet, dass** sie mindestens ein Magnesiumsalz enthält.

4. Topische Zusammensetzung nach einem der Ansprüche 1 bis 2, **dadurch gekennzeichnet, dass** mindestens ein Calciumsalz enthält.

5. Topische Zusammensetzung nach einem der Ansprüche 1 bis 2, **dadurch gekennzeichnet, dass** sie einen Verbund von Magnesiumsalz und Calciumsalz umfasst.

6. Topische Zusammensetzung nach Anspruch 5, **dadurch gekennzeichnet, dass** sie einen Verbund von 1/2 Magnesiumlactat und 1/2 Calciumlactat bis zu einem Verbund von 1/4 Magnesiumlactat und 3/4 Calciumlactat, also einem Verhältnis von 1 zu 1 bis 1 zu 3, bezogen auf das Gesamtgewicht der Zusammensetzung, umfasst.

7. Topische Zusammensetzung nach Anspruch 6, **dadurch gekennzeichnet, dass** sie einen Verbund von Magnesiumsalz und Calciumsalz in einem Anteil von 2/3 // 1/3, also einem Verhältnis von 2 zu 1, bezogen auf das Gewicht, umfasst.

8. Topische Zusammensetzung nach einem der vorhergehenden Ansprüche, **dadurch gekennzeichnet, dass** der Blaualgenextrakt, der in der Zusammensetzung vorliegt, zwischen 0,001 Gew.-% und 0,01 Gew.-% des Gesamtgewichts der Zusammensetzung ausmacht.

9. Topische Zusammensetzung nach einem der vorhergehenden Ansprüche, **dadurch gekennzeichnet, dass** das alpha-Hydroxysäuresalz oder die alpha-Hydroxysäuresalze, das bzw. die in der Zusammensetzung vorliegt bzw. vorliegen, zusammen oder jedes getrennt betrachtet, zwischen 0,001 Gew.-% und 0,01 Gew.-% des Gesamtgewichts der Zusammensetzung ausmacht bzw. ausmachen.

10. Topische Zusammensetzung nach einem der Ansprüche 1 bis 9, **dadurch gekennzeichnet, dass** sie in der Form eines Produkts zur Pflege und/oder zum Schminken von Körper- oder Gesichtshaut, Lippen, Wimpern, Augenbrauen, Haaren, Kopfhaut, Nägeln oder Schleimhäuten; eines Sonnenschutz- oder Selbstbräunungsprodukts; eines Haarbehandlungs-, insbesondere Färbungs-, Conditioner- und/oder Haarpflegeprodukts dargereicht wird.

11. Topische Zusammensetzung nach einem der Ansprüche 1 bis 9, **dadurch gekennzeichnet, dass** sie mindestens eine flüssige Phase umfasst, die mindestens eine Verbindung umfasst, die aus Ölen und/oder Lösungsmitteln mit mineralischem, tierischem, pflanzlichem oder synthetischem Ursprung, kohlenstoff-, kohlenwasserstoff-, fluor- und/oder silikonhaltigen, salzhaltigen, flüchtigen oder nichtflüchtigen Ölen und/oder Lösungsmitteln, allein oder im Gemisch, ausgewählt ist.

12. Kosmetische Verwendung einer topischen Zusammensetzung nach einem der Ansprüche 1 bis 11 bei der Verhinderung und/oder der Behandlung von Anzeichen, die mit der Alterung eines Lebewesens verbunden sind, insbesondere jenen, die sich auf Haut und/oder Hautanhangsgebilde und/oder Schleimhäute auswirken.

13. Kosmetische Verwendung nach Anspruch 12 bei der Verhinderung von Anzeichen der Hautalterung.

14. Topische Zusammensetzung nach einem der Ansprüche 1 bis 9 zu deren Verwendung bei der Verhinderung und/oder der Behandlung von Leiden der Mundschleimhaut.

15. Verfahren zur kosmetischen Behandlung von Keratinsubstanzen, insbesondere Körper- oder Gesichtshaut, Lippen, Nägeln, Haaren, Wimpern und/oder Schleimhäuten, das die Anwendung einer wie in einem der Ansprüche 1 bis 9 definierten kosmetischen Zusammensetzung auf die besagten Substanzen umfasst.

16. Verfahren zur Herstellung von Zusammensetzung nach den Ansprüchen 1 bis 11, **dadurch gekennzeichnet, dass** es die folgenden Schritte umfasst:

    1- Erhalten der Blaualgenbiomasse:
    -- die Biomasse wird nach einer Kultivierungsdauer von 10 bis 20 Tagen unter adäquaten Temperatur- und Beleuchtungsbedingungen geerntet,
    2- Erhalten des Blaualgenextrakts:
    -- die Biomasse wird gewaschen und dann fein gemahlen, die wässrige Phase wird durch Zentrifugation oder Filtration von Zelltrümmern getrennt,
    3- der so erhaltenen Lösung wird Folgendes zugegeben:

        -- alpha-Hydroxysäure, das aus Magnesium-, Calciumand deren Gemischen ausgewählt ist
        -- Maltodextrin, das als Träger dient,

    4- der pH-Wert der Proben wird unter Verwendung von NaOH (0,1 N) auf pH = 6,5 zurückgebracht,
    5- das trockene Produkt wird durch Entfernen von Wasser durch eine geeignete Technik erhalten, bei der es sich beispielsweise um folgende handeln kann: ein Lyophilisations-, Zerstäubungs- oder Trocknungsverfahren in einem belüfteten Trockenofen bei niedriger Temperatur.

## Claims

1. Topical composition **characterised in that** it contains at least one blue algae extract of the *Phormidium sp* type and at least one alpha hydroxyl acid salt soluble in an aqueous medium, chosen from the salts of magnesium, and of calcium as well as mixtures thereof, in combination with at least one excipient acceptable for topical application.

2. Topical composition according to claim 1, **characterised in that** said alpha hydroxyl acid salt is a lactate.

3. Topical composition according to one of claims 1 to 2, **characterised in that** it contains at least one magnesium salt.

4. Topical composition according to one of claims 1 to 2, **characterised in that** it contains at least one calcium salt.

5. Topical composition according to one of claims 1 to 2, **characterised in that** it comprises a combination of magnesium salt and of calcium salt.

6. Topical composition according to claim 5, **characterised in that** it comprises a combination ranging from 1/2 magnesium lactate and 1/2 calcium lactate to a combination of 1/4 magnesium lactate and 3/4 calcium lactate i.e. a ratio of 1 for 1 to 1 for 3 by total weight of the composition.

7. Topical composition according to claim 6, **characterised in that** it comprises a combination of magnesium salt and of calcium salt in a proportion of 2/3 // 1/3 i.e. a ratio of 2 for 1 by weight.

8. Topical composition as claimed in any preceding claim, **characterised in that** the blue algae extract present in the composition represents between 0.001% and 0.01% by weight, of the total weight of the composition.

9. Topical composition as claimed in any preceding claim, **characterised in that** the alpha hydroxyl acid salt or salts present in the composition represent, together or each one considered separately, between 0.001% and 0.01% by weight, of the total weight of the composition.

10. Topical composition according to one of claims 1 to 9, **characterised in that** it has the form of a care and/or makeup product of the skin of the body of or the face, of the lips, of the eyelashes, of the eyebrows, of the hair, of the scalp, of the nails or of the mucosa; of a solar or self-tanning product; of a hair product in particular for colouring, conditioning and/or treating hair.

11. Topical composition according to one of claims 1 to 9, **characterised in that** it comprises at least one liquid phase comprising at least one compound chosen from oils and/or solvents of mineral, animal, plant or synthetic origin, carbonated, hydrocarbonated, fluorinated and/or siliconised, serum, volatile or nonvolatile, alone or in a mixture.

12. Cosmetic use of a topical composition according to one of claims 1 to 11, in the prevention and/or the treatment of all of the signs linked to the ageing of the organism in particular those that affect the skin and/or the appendages and/or the mucosa.

13. Cosmetic use according to claim 12, in the prevention of the signs of cutaneous ageing.

14. Topical composition according to one of claims 1 to 9 for the use thereof in the prevention and/or the treatment of the affections of the oral mucosa.

15. Method of cosmetic treatment for keratinous materials, in particular the skin of the body or of the face, of the lips, of the nails, of the hair, of the eyelashes and/or of the mucosa, comprising the application on said materials of a cosmetic composition such as defined in one of claims 1 to 9.

16. Method for the preparation of compositions according to claims 1 to 11 **characterised in that** it comprises the following steps:

   1-Obtaining biomass from blue algae:
   -- The biomass is collected after a duration of culture from 10 to 20 days in adequate temperature and light conditions,
   2-Obtaining blue algae extract:
   -- The biomass is washed and then finely ground. The aqueous fraction is separated from the cellular debris by centrifugation or filtration,
   3-To the solution obtained as such is added:

      -- alpha hydroxy acid chosen from the salts of magnesium, of calcium as well as mixtures thereof
      -- maltodextrin serving as a support

   4-The pH of the samples is brought to pH=6.5 by using NaOH (0.1N),
   5-The dry product is obtained by removing water with a suitable technique, which can for example be: a method of lyophilisation, spraying or drying in a low-temperature ventilated oven.

Figure 1

Figure 2

Figure 3

CD44

Témoin

Cyano+ Lactates
1 mg/ml

# Figure 4

Figure 5

| | Cyano + Lactates | |
|---|---|---|
| | 50 µg/ml | 100 µg/ml |
| **Collagène I** | +196 % | + 25% |
| **Elastine** | +177 % | +37 % |

# Figure 6

|  |  | MMP-1 | MMP-3 | MMP-12 |
|---|---|---|---|---|
| Témoin P7 | Fibroblaste jeunes | 100 | 100 | 100 |
| Témoin P17 | Fibroblastes âgés | 2128% | 551% | 1374% |
| Témoin P17 : Lact+Cyano (10μg/ml) | Fibroblastes âgés | -55% | -49% | -46% |

# Figure 7

**EP 2 785 314 B2**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 1345584